# EUROPEAN PATENT SPECIFICATION

(11) **EP 0 691 850 B1**
(45) Date of publication and mention of the grant of the patent: **25.09.2002**
(21) Application number: 94908476.8
(22) Date of filing: 02.03.1994
(51) Int. Cl.: A61K 38/36, A61K 38/55, A61K 9/00, A61P 17/02

(54) **FACTOR XIII FOR TREATMENT OF SKIN WOUNDS**
Faktor XIII zur Behandlung von Hautwunden
FACTEUR XIII POUR LE TRAITEMENT DES LESIONS CUTANEES

(30) Priority: 30.03.1993 JP 7139593
(43) Date of publication of application: 17.01.1996
(73) Proprietor: HOECHST JAPAN LIMITED, Tokyo 107 (JP)
(72) Inventor: KATO, Naoko, Hiki-gun, Saitama 350-03 (JP); OGAWA, Takashi, Sakado-shi, Saitama 350-02 (JP); KONDO, Shuji, Kokubunji-shi, Tokyo 185 (JP)
(74) Representative: Lauppe, Hans Friedrich, Dr.
(86) International application number: IB9400040
(87) International publication number: WO94022470

(56) References cited:
- EP-A- 0 268 772
- EP-A- 0 332 985
- EP-A- 0 358 995
- FR-A- 2 422 407
- GB-A- 2 042 556
- THE JAPANESE JOURNAL OF PHARMACOLOGY vol. 61 , 1993 , KYOTO page 346P TAKASHI OGAWA ET AL 'Relatioship between factor XIII and delayed burn healing in CCl4-treated rats.'

## Description

The present invention relates to an external preparation for treatment of skin wounds containing as an active ingredient human blood coagulation factor XIII.

Human coagulation factor XIII has been known in the past as a therapeutic agent for skin wounds, and has been administered intravenously. However, there are problems with this administration route regarding delivery of the drug to the wound site. In addition, this administration requires the attendance of a physician.

In EP-A-0 358 995 it is disclosed that factor XIII can be used in a process for preparing a topical medicament for immunsuppression.

It is an object of the present invention to provide a human coagulation factor XIII preparation for skin treatment that is free of the problems of the prior art mentioned above.

The present invention provides a dermal preparation for treatment of skin wounds containing as an active ingredient human blood coagulation factor XIII. It is preferable that the dermal preparation of the present invention contain aprotinin as well. The dermal preparation of the present invention is particularly suitable for local treatment of bums. Human blood coagulation factor XIII of human plasma or placenta origin is used preferably, and that produced by recombinant gene technology can also be used.

Human blood coagulation factor XIII (hereinafter referred to as factor XIII) is widely found in plasma, placenta and the like and activated with thrombin and Ca²⁺. The activated factor XIII catalyzes ξ-(γ-glutamyl)lysil cross-links between specific protein pairs. The cross-linking between fibrin monomers to form γ-γ dimers and α-chains to form α-polymers by activated factor XIII could provide fibrin with strength and elasticity and act on hemostatic mechanism. Furthermore, factor XIII catalyzes crosslinking of fibronectin to an α-chain of fibrin or collagen, and thus plays an important role in the process of wound healing [Matsuda, Acta Haematologica Japonica (in Japanese)(1977), Vol. 40, No. 6, pp. 995-1002].

Factor XIII preparation has been intravenously administered mainly for the treatment of wound healing disturbances; now, the present inventors have made various studies and, as a result, found that factor XIII could percutaneously penetrate wounded skin and permeate into the affected skin tissue in a sufficient amount to be required for the treatment. The present invention has been completed to prove that factor XIII is effective as a dermal agent as well.

Factor XIII preparation does not cause any troubles in side-effects, toxicity and others usually at a dose of 20-50 units/kg/body/day, in view of the results of acute toxicity test as described below and intravenous applications of over 10,000 cases at home and abroad.

As a dermal agent, one may administer the agent at 1-1000 units/site to be given/body/day, depending upon the severity of disturbance at the damaged portion and the damaged stage. One unit of factor XIII as used herein is defined as an amount of factor XIII contained in 1 ml of plasma of healthy person, which is usually about 15 µg/ml.

The results of acute toxicity test of factor XIII are shown in Table 1.

**Table 1**

| **Animal species** | **Administration route** | **Sex** | **LD**_{**50**} **(units/kg)** |
|---|---|---|---|
| Mice (NMRI strain) Male and female, each 10 animals/group | i.v. | Male Female | >3,125 |
| Rats (Wistar strain) Male and female, each 10 animals/group | i.v. | Male Female | >625 |

The dermal agent of the present invention is applicable to treatment of a variety of skin wounds such as cut wound, stabbed wound, cracked wound, contused wound, lacerated wound, decupitus, bum and the like and is particularly effective for local treatment of bum.

Factor XIII preparation may be prepared by fractional purification of plasma starting from human placenta or plasma according to a well-known method in the art. The preparation as prepared by fractional purification of plasma might contain hepatitis virus, AIDS virus and others and such viruses should be inactivated by heat treatment and other means. Heat treatment is performed by dissolving factor XIII in an aqueous solution of sodium chloride containing EDTA and heating the solution at about 60°C for approximately 10 hours.

Factor XIII may be also prepared according to genetic engineering techniques. Factor XIII which is employed in the present invention may include those factors prepared by all available methods including not only fractional purification but also genetic engineering techniques and others.

As the dosage forms of the present dermal agent, there may be mentioned watersoluble ointments, oleaginous ointments, lotions, sprays, oils, gels and the like. As representative bases, there may be mentioned macrogol bases for watersoluble ointments, petrolatum bases for oleaginous ointments, olive oil, sesame oil, camellia oil and the like for oils, carboxyvinyl polymers, sodium polyacrylate and the like for gels.

Factor XIII is, for instance, dissolved in an aqueous solution of sodium chloride containing glucose, human serum albumin and the like, aseptically filtered after its titer is adjusted, poured into vials and then freeze-dried. At the time of use, it is reconstituted in distilled water for injection and the resultant solution as such is used as solutions or filled into a vessel equipped with a spray device and then used as sprays.

As assays for factor XIII activity, there are employed a fibrin-formation method, a transglutaminase activity assay, an immunological assay for antigen level and others. There was employed in Examples of the present invention the dansyl-cadaverine method by Y. Nishida et al., which can determine an amount of activated factor XIII with a high sensitivity [Y. Nishida et al., (1984), Thromb. Res., Vol. 36, pp. 123-131]. Dansylcadaverine (available from IATRON Laboratories, Inc.) is a fluorescent amine which may be a substrate for factor XIII, it may form a dansylcadaverine-casein complex with casein by the action of factor XIII and, after reaction, fluorescence intensity of the said complex recovered by gel filtration may be measured to determine the factor XIII activity.

This invention will be more illustratively explained by way of the following Production Example, Preparation Examples and Test Examples.

### Examples:

### Production Example 1 - Production of factor XIII from human placenta

Placenta was freezed, finely divided, stirred with an aqueous solution of sodium chloride and then centrifuged to collect supernatant I. The supematant I was confirmed to be negative for HBs antigen by enzyme immunoassay and an acrinol (Rivanol) solution was added to form precipitate II, which was then washed and stirred with an aqueous solution of sodium chloride containing EDTA. After removing undissolved substance (precipitate III), to the resultant supematant III was added an N-cetyl-pyridinium chloride solution to precipitate concomitant proteins and mucopolysaccharides and then an acrinol solution was added to supematant IV to form a precipitate V containing factor XIII. To the precipitate V was added an aqueous solution of sodium chloride containing EDTA. After stirring, to supernatant VI, from which undissolved substance (precipitate VI) was removed, was added ammonium sulfate to form a precipitate VII containing factor XIII. To the precipitate VII was added an EDTA solution and the resulting mixture was dialyzed against a Tris-hydrochloride buffer containing EDTA and sodium azide. After pH was adjusted, the precipitate VIII thus formed was removed, supernatant VIII was gel-filtrated to collect active fractions. To the fractions was added ammonium sulfate to form a precipitate IX containing factor XIII. The precipitate IX was dissolved in a Tris-hydrochloride buffer containing EDTA, dialyzed against the same buffer and pH was adjusted to precipitate factor XIII as an euglobulin. This euglobulin precipitate was dissolved in an aqueous solution of sodium chloride and then aminoacetic acid and sucrose were added. Then, ammonium sulfate was added to form a precipitate X containing factor XIII, which was dissolved in an aqueous solution of sodium chloride containing EDTA and dialyzed against the same solution. Thereafter, the titer of factor XIII was adjusted using an aqueous solution of sodium chloride containing glucose and human serum albumin.

### Preparation Example 1 - Preparation 1 of a therapeutic agent for dermal use containing factor XIII

An dermal preparation was prepared using the factor XIII solution prepared as described in the above Production Example 1. More specifically, after factor XIII was adjusted to a titer of 240 units/ml and filtered aseptically, the so adjusted factor XIII was transferred to a glass vial together with 32 mg of human serum albumin, 20 mg of glucose and 34 mg of sodium chloride, followed by freeze drying. At the time of use, this freeze-dried factor XIII was dissolved with distilled water for injection so that the concentration of factor XIII at administration was 120 units/ml. This solution was used as a spray solution.

### Preparation Example 2 - Preparation 2 of a therapeutic agent for dermal use containing factor XIII

The freeze-dried factor XIII identical to that prepared in Preparation Example 1 was dissolved in an aprotinin solution having a concentration of 1000 units/ml or more at the time of use so that the concentration of factor XIII was 120 units/ml. This solution was used as a spray solution.

### Test Example 1 - In Vitro Diffusion Cell Study

The abdominal skin was excised from Sprague-Dawley rats (males, 9-10 weeks old, body weight: 300 g) after shaving, or heating for 45 seconds in a 60°C water bath after shaving, and sandwiched between a lid and a receptor chamber for measurement of percutaneous absorption having a surface area of approximately 8 cm² (John J. Windheuser, et al. (1982), J. of Pharmaceutical Sciences, Vol. 71, No. 11, pp. 1211-1213). The preparation of Preparation Example 1 or 2 (concentration: 120 units/ml) was applied in an amount of 1 ml to the upper surface of the skin specimens. The lower surface of each skin specimen was brought into contact in advance with 45 ml of 0.05 M Tris-HCl·0.15 M NaCI solution (pH 7.5). The cells were maintained at 37°C to observe percutaneous absorption for 8 hours. During the period, 20 µl samples were withdrawn from the receptor solution through the side arm every 2 hours to determine the activity of factor XIII in those samples. The results for the skin excised after shaving are shown in Table 2, while the results for the skin heat treated after shaving are shown in Table 3, provided that the total amount of the preparation applied to the skin upper surface is 100%.

**Table 2**

| | Cumulative Value of Activity of Preparation Permeated Skin (%) | | | |
|---|---|---|---|---|
| Preparation | After 2 hrs. | After 4 hrs. | After 6 hrs. | After 8 hrs. |
| Preparation Example 1 | 0.2 | 0.3 | 0.2 | 0.3 |
| Preparation Example 2 | 0.4 | 0.5 | 0.5 | 0.5 |
| (n=3) | | | | |

**Table 3**

| | Cumulative Value of Activity of Preparation Permeated Skin (%) | | | |
|---|---|---|---|---|
| Preparation | After 2 hrs. | After 4 hrs. | After 6 hrs. | After 8 hrs. |
| Preparation Example 1 | 0.7 | 5.9 | 9.5 | 12.5 |
| Preparation Example 2 | 3.2 | 9.8 | 16.6 | 23.0 |
| (n=3) | | | | |

When the skin excised after shaving was used, factor XIII had hardly permeated the skin in the case of both Preparation Example 1 and Preparation Example 2. However, when the skin that had been burned by heat treatment was used, factor XIII permeated well the skin both in the case of Preparation Example 1 and Preparation Example 2. Preparation Example 2 containing aprotinin showed particularly excellent permeation.

### Test Example 2 - Rat Burn Model Administration Test

The dorsal skin of Sprague-Dawley rats (males, 9-10 weeks old, body weight: 300 g) was shaved, and a metal rod heated to 85°C (70 g, diameter: 18 mm) was pressed to the skin for 20 seconds under nembutal anesthesia to provide bum models. Two bum models were formed on both sides of the backbone of each animal. The preparation of Preparation Example 2 was sprayed in an amount of 30 units/0.2 ml onto the surface of the burned skin each day. Specimens of burned skin having a surface area of approximately 2 cm² were then excised after 2 days, washed with physiological saline solution and homogenized after addition of 0.05 M Tris-HCl·0.15 M NaCI solution (pH 7.5). After centrifugation of the homogenate, the activity of factor XIII in the supematant was determined. As a control, 120 units of the preparation were administered intravenously, and its dorsal skin having the same area as that of the burned skin was excised after 2 days. The specimen was washed with physiological saline solution and homogenized after addition of 0.05 M Tris-HCl· 0.15 M NaCI solution (pH 7.5). After centrifugation of the homogenate, the activity of factor XIII in the supematant was determined. As a result, the activity of factor XIII per 1 cm² of skin is shown in Table 4 in terms of the unit of factor XIII defined above.

**Table 4**

| Administration Method | Factor XIII Activity in Skin (units/cm²) |
|---|---|
| Spray Administration | 0.050 |
| Intravenous Administration | 0.023 |
| (n=4) | |

According to the results shown in Table 4, although the total dose in the case of spray administration is 1/2 that of intravenous administration, the factor XIII activity in the skin was roughly 2 times greater. This result suggests that it is more effective to administer the factor XIII preparation in the form of an dermal preparation rather than intravenous administration in terms of treatment of wounds.

### Production Example 2 - Expression of recombinant factor XIII

The amino acid sequence of the recombinant factor XIII used in the present invention is that of human factor XIII from the first position, Ser, to the 731st position, Met,
and Phe is substituted for Leu in the 88th position in Figure 3 of Grundmann (U. Grundmann, et al. (1986), Proc. Natl. Acad. Sci. USA, Vol. 83, pp. 8024-8028. The DNA sequence encoding the amino acid sequence described above was inserted into the region from Sstl to HindIII of the polylinker site of pEMBLyex4 (J. K. Selton, Genetic Engineering (1987), Plenum Publishing Co., Vol. 9, pp. 135-154) that is an expression vector for yeast, which was used as a plasmid for production of factor XIII. The yeast host cell, CL3ABYS86 (Offenlegungsshrift DE 3804890 A1), was transfected by the above plasmid to produce recombinant factor XIII in yeast cells. Then the recombinant factor XIII was purified from the supematant of yeast cell destruction solution.

### Preparation Example 3 - Preparation 3 of a therapeutic agent for dermal use containing factor XIII

The recombinant factor XIII solution produced in the above Production Example 2 was diluted with a solution containing 1.6 % of human serum albumin, 1 % of glucose and 1.7% of sodium chloride so that the concentration of factor XIII at administration is 120 units/ml. This solution was used as a spray solution.

### Test Example 3 - In Vitro Diffusion Cell Study

The abdominal skin was excised from Sprague-Dawley rats (males, 9-10 weeks old, body weight: 300 g) after shaving, or heating for 45 seconds in a 60°C water bath after shaving, and sandwiched between a lid and a receptor chamber for measurement of percutaneous absorption having a diameter of 7 mm (above described in Test Example 1). The preparation of Preparation Example 1 or 3 ( concentration: 120 units/ml) was applied in an amount of 0.1 ml to the upper surface of the skin specimens. The lower surface of each skin specimen was brought into contact in advance with 4.7 ml of 0.05 M Tris-
HCI·0.15M NaCI solution (pH 7.5). The cells were maintained at 37°C to observe percutaneous absorption for 8 hours. During the period, 20 µl samples were withdrawn from the receptor solution through the side arm every 2 hours to determine the activity of factor XIII in those samples. The results for the skin excised after shaving are shown in Table 5, while the results for the skin heat treated after shaving are shown in Table 6, provided that the total amount of the preparation applied to the skin upper surface is 100%.

**Table 5**

| | Cumulative Value of Activity of Preparation Permeated Skin (%) | | | |
|---|---|---|---|---|
| Preparation | After 2 hrs. | After 4 hrs. | After 6 hrs. | After 8 hrs. |
| Preparation Example 1 | 0.1 | 0.2 | 0 | 0 |
| Preparation Examples | 0.3 | 0.3 | 0.3 | 0.5 |
| (n=3) | | | | |

**Table 6**

| | Cumulative Value of Activity of Preparation Permeated Skin (%) | | | |
|---|---|---|---|---|
| Preparation | After 2 hrs. | After 4 hrs. | After 6 hrs. | After 8 hrs. |
| Preparation Example 1 | 0.1 | 1.2 | 4.3 | 7.9 |
| Preparation Example 3 | 0.3 | 3.2 | 6.5 | 12.7 |
| (n=3) | | | | |

The results above show that the dermal preparation containing the recombinant factor XIII is as effective as that containing factor XIII of human placenta origin.

When the dermal preparation of the present invention is applied to the wounded skin, particularly to bums or scalds, the active ingredient human blood coagulation factor XIII shows excellent drug delivery to the wound compared with the case of intravenous administration. In addition, the preparation of the present invention can be administered without assistance of a physician.

## Claims

1. Pharmaceutical agent for the topical treatment of skin wounds, containing as pharmaceutically active ingredients only human coagulation factor XIII and aprotinin.

2. The pharmaceutical agent of claim 1, wherein the coagulation factor is derived from human plasma.

3. The pharmaceutical agent of claim 1, wherein the coagulation factor is derived from human placenta.

4. The pharmaceutical agent of claim 1, wherein the coagulation factor is produced by genetic engineering.

5. The pharmaceutical agent of claim 4, wherein the coagulation factor is a physiologically active mutant of factor XIII.

6. The pharmaceutical agent of any of claims 1 to 5 topically administered to a wound.

7. The pharmaceutical agent of any of claims 1 to 5 topically administered to a wound caused by bum.

8. The pharmaceutical agent of any of claims 1 to 5 topically administered to a wound caused by decubitus.

## Patentansprüche

1. Pharmazeutisches Mittel für die topische Behandlung von Hautwunden, welches als pharmazeutisch wirksame Bestandteile nur Human-Gerinnungsfaktor XIII und Aprotinin enthält.

2. Pharmazeutisches Mittel nach Anspruch 1, wobei der Gerinnungsfaktor aus menschlichem Plasma stammt.

3. Pharmazeutisches Mittel nach Anspruch 1, wobei der Gerinnungsfaktor aus menschlicher Plazenta stammt.

4. Pharmazeutisches Mittel nach Anspruch 1, wobei der Gerinnungsfaktor mittels Genmanipulation hergestellt wird.

5. Pharmazeutisches Mittel nach Anspruch 1, wobei der Gerinnungsfaktor eine physiologisch wirksame Mutante von Faktor XIII ist.

6. Pharmazeutisches Mittel nach einem der Ansprüche 1 bis 5, topisch verabreicht an eine Wunde.

7. Pharmazeutisches Mittel nach einem der Ansprüche 1 bis 5, topisch verabreicht an eine durch Verbrennung hervorgerufene Wunde.

8. Pharmazeutisches Mittel nach einem der Ansprüche 1 bis 5, topisch verabreicht an eine durch einen Dekubitus hervorgerufene Wunde.

## Revendications

1. Agent pharmaceutique pour le traitement local de lésions cutanées, contenant comme composants pharmaceutiquement actifs seulement du facteur de coagulation XIII humain et de l'aprotinine.

2. Agent pharmaceutique de la revendication 1, dans lequel le facteur de coagulation est issu de plasma humain.

3. Agent pharmaceutique de la revendication 1, dans lequel le facteur de coagulation est issu de placenta humain.

4. Agent pharmaceutique de la revendication 1, dans lequel le facteur de coagulation est produit par génie génétique.

5. Agent pharmaceutique de la revendication 4, dans lequel le facteur de coagulation est un mutant physiologiquement actif du facteur XIII.

6. Agent pharmaceutique de l'une quelconque des revendications 1 à 5, appliqué localement sur une lésion.

7. Agent pharmaceutique de l'une quelconque des revendications 1 à 5, appliqué localement sur une lésion provoquée par une brûlure.

8. Agent pharmaceutique de l'une quelconque des revendications 1 à 5, appliqué localement sur une lésion provoquée par décubitus.
